# EUROPEAN PATENT SPECIFICATION

(11) **EP 0 699 075 B1**
(45) Date of publication and mention of the grant of the patent: **12.12.2001**
(21) Application number: 94918092.1
(22) Date of filing: 23.05.1994
(51) Int. Cl.: A61K 38/48

(54) **MODIFIED FACTOR VII for inhibiting vascular restenosis and platelet deposition**
MODIFIZIERTER FAKTOR VII zur Hemmung der vaskulären Restenosis und Thrombozytenablagerung
FACTEUR VII MODIFIE pour l'inhibition de la resténose vasculaire et du dépôt des plaquettes

(30) Priority: 21.05.1993 US 65725
(43) Date of publication of application: 06.03.1996
(73) Proprietor: ZYMOGENETICS, INC., Seattle, WA 98105 (US); NOVO NORDISK A/S, 2880 Bagsvaerd (DK)
(72) Inventor: BERKNER, Kathleen, L., Seattle, WA 98199 (US); PETERSEN, Lars, Christian, DK-Hoersholm (DK); HART, Charles, E., Brier, WA 98036 (US)
(74) Representative: Jones, Alan John
(86) International application number: US9405779
(87) International publication number: WO9427631

(56) References cited:
- WO-A-91/11514
- WO-A-92/15686
- J. Intervent. Cardiol., 1993, 6: 187-202. Anderson et al.: Restenosis after coronary angioplasty
- Hirsh et al.: Overview of the thrombotic process and its therapy in hemostasis and thrombosis, Basic Principles and Clinical Practice, Eds. Colman et al., Lipincott Company, Philadelphia, PA,1994, pages 1151-1163

### SEQUENCE LISTING

(1) GENERAL INFORMATION:
   (i) APPLICANT:
      (A) NAME: ZymoGenetics, Inc. et al.
      (B) STREET: 4225 Roosevelt Way N.E.
      (C) CITY: Seattle
      (D) STATE: Washington
      (E) COUNTRY: United States of America
      (F) POSTAL CODE (ZIP): 98105
      (G) TELEPHONE: (206)547-8080
      (H) TELEFAX:
      (I) TELEX:
   (ii) TITLE OF INVENTION: Modified Factor VII
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER:
      (B) FILING DATE: 23-MAY-1994
      (C) CLASSIFICATION:
   (vi) PRIOR APPLICATION DATA:
      (A) APPLICATION NUMBER: US 08/065,725
      (B) FILING DATE: 21-MAY-1993
   (vi) PRIOR APPLICATION DATA:
      (A) APPLICATION NUMBER: US 07/662,920
      (B) FILING DATE: 28-FEB-1991
   (vii) ATTORNEY/AGENT INFORMATION:
      (A) NAME: Parmelee, Steven W.
      (B) REGISTRATION NUMBER: 31,990
      (C) REFERENCE/DOCKET NUMBER: 13952-8-1PC
   (viii) TELECOMMUNICATION INFORMATION:
      (A) TELEPHONE: 206-467-9600
      (B) TELEFAX: 415-543-5043
(2) INFORMATION FOR SEQ ID NO:1:
   (i) SEQUENCE CHARACTERISTICS:
      (A) LENGTH: 2422 base pairs
      (B) TYPE: nucleic acid
      (C) STRANDEDNESS: single
      (D) TOPOLOGY: linear
   (ii) MOLECULE TYPE: CDNA
   (iii) HYPOTHETICAL: N
   (iv) ANTI-SENSE: N
   (ix) FEATURE:
      (A) NAME/KEY: CDS
      (B) LOCATION: 28..1420
      (D) OTHER INFORMATION: /codon_start= 28
         /product= "Factor VII"
   (xi) SEQUENCE DESCRIPTION: SEQ ID NO:1:
   CCTCCCGACA ATACAGGGGC AGCACTGCAG AGATTTCATC ATG GTC TCC CAG GCC    55
                                               Met Val Ser Gln Ala
                                               -38         -35
   CTC AGG CTC CTC TGC CTT CTG CTT GGG CTT CAG GGC TGC CTG GCT GCA   103
   Leu Arg Leu Leu Cys Leu Leu Leu Gly Leu Gln Gly Cys Leu Ala Ala
               -30                 -25                 -20
   GTC TTC GTA ACC CAG GAG GAA GCC CAC GGC GTC CTG CAC CGG CGC CGG   151
   Val Phe Val Thr Gln Glu Glu Ala His Gly Val Leu His Arg Arg Arg
           -15                 -10                 -5
   CGC GCC AAC GCG TTC CTG GAG GAG CTG CGG CCG GGC TCC CTG GAG AGG   199
   Arg Ala Asn Ala Phe Leu Glu Glu Leu Arg Pro Gly Ser Leu Glu Arg
        1               5                  10                  15
   GAG TGC AAG GAG GAG CAG TGC TCC TTC GAG GAG GCC CGG GAG ATC TTC   247
   Glu Cys Lys Glu Glu Gln Cys Ser Phe Glu Glu Ala Arg Glu Ile Phe
                    20                  25                  30
   AAG GAC GCG GAG AGG ACG AAG CTG TTC TGG ATT TCT TAC AGT GAT GGG   295
   Lys Asp Ala Glu Arg Thr Lys Leu Phe Trp Ile Ser Tyr Ser Asp Gly
               35                  40                  45
   GAC CAG TGT GCC TCA AGT CCA TGC CAG AAT GGG GGC TCC TGC AAG GAC   343
   Asp Gln Cys Ala Ser Ser Pro Cys Gln Asn Gly Gly Ser Cys Lys Asp
           50                  55                  60
   CAG CTC CAG TCC TAT ATC TGC TTC TGC CTC CCT GCC TTC GAG GGC CGG   391
   Gln Leu Gln Ser Tyr Ile Cys Phe Cys Leu Pro Ala Phe Glu Gly Arg
       65                  70                  75
   AAC TGT GAG ACG CAC AAG GAT GAC CAG CTG ATC TGT GTG AAC GAG AAC   439
   Asn Cys Glu Thr His Lys Asp Asp Gln Leu Ile Cys Val Asn Glu Asn
   80                  85                  90                  95
   GGC GGC TGT GAG CAG TAC TGC AGT GAC CAC ACG GGC ACC AAG CGC TCC   487
   Gly Gly Cys Glu Gln Tyr Cys Ser Asp His Thr Gly Thr Lys Arg Ser
                   100                 105                 110
   TGT CGG TGC CAC GAG GGG TAC TCT CTG CTG GCA GAC GGG GTG TCC TGC   535
   Cys Arg Cys His Glu Gly Tyr Ser Leu Leu Ala Asp Gly Val Ser Cys
               115                 120                 125
   ACA CCC ACA GTT GAA TAT CCA TGT GGA AAA ATA CCT ATT CTA GAA AAA   583
   Thr Pro Thr Val Glu Tyr Pro Cys Gly Lys Ile Pro Ile Leu Glu Lys
           130                 135                 140
   AGA AAT GCC AGC AAA CCC CAA GGC CGA ATT GTG GGG GGC AAG GTG TGC   631
   Arg Asn Ala Ser Lys Pro Gln Gly Arg Ile Val Gly Gly Lys Val Cys
       145                 150                 155
   CCC AAA GGG GAG TGT CCA TGG CAG GTC CTG TTG TTG GTG AAT GGA GCT   679
   Pro Lys Gly Glu Cys Pro Trp Gln Val Leu Leu Leu Val Asn Gly Ala
   160                 165                 170                 175
   CAG TTG TGT GGG GGG ACC CTG ATC AAC ACC ATC TGG GTG GTC TCC GCG   727
   Gln Leu Cys Gly Gly Thr Leu Ile Asn Thr Ile Trp Val Val Ser Ala
                   180                 185                 190
   GCC CAC TGT TTC GAC AAA ATC AAG AAC TGG AGG AAC CTG ATC GCG GTG   775
   Ala His Cys Phe Asp Lys Ile Lys Asn Trp Arg Asn Leu Ile Ala Val
               195                 200                 205
   CTG GGC GAG CAC GAC CTC AGC GAG CAC GAC GGG GAT GAG CAG AGC CGG   823
   Leu Gly Glu His Asp Leu Ser Glu His Asp Gly Asp Glu Gln Ser Arg
           210                 215                 220
   CGG GTG GCG CAG GTC ATC ATC CCC AGC ACG TAC GTC CCG GGC ACC ACC    871
   Arg Val Ala Gln Val Ile Ile Pro Ser Thr Tyr Val Pro Gly Thr Thr
       225                 230                 235
   AAC CAC GAC ATC GCG CTG CTC CGC CTG CAC CAG CCC GTG GTC CTC ACT    919
   Asn His Asp Ile Ala Leu Leu Arg Leu His Gln Pro Val Val Leu Thr
   240                 245                 250                 255
   GAC CAT GTG GTG CCC CTC TGC CTG CCC GAA CGG ACG TTC TCT GAG AGG    967
   Asp His Val Val Pro Leu Cys Leu Pro Glu Arg Thr Phe Ser Glu Arg
                   260                 265                 270
   ACG CTG GCC TTC GTG CGC TTC TCA TTG GTC AGC GGC TGG GGC CAG CTG   1015
   Thr Leu Ala Phe Val Arg Phe Ser Leu Val Ser Gly Trp Gly Gln Leu
               275                 280                 285
   CTG GAC CGT GGC GCC ACG GCC CTG GAG CTC ATG GTC CTC AAC GTG CCC   1063
   Leu Asp Arg Gly Ala Thr Ala Leu Glu Leu Met Val Leu Asn Val Pro
           290                 295                 300
   CGG CTG ATG ACC CAG GAC TGC CTG CAG CAG TCA CGG AAG GTG GGA GAC   1111
   Arg Leu Met Thr Gln Asp Cys Leu Gln Gln Ser Arg Lys Val Gly Asp
       305                 310                 315
   TCC CCA AAT ATC ACG GAG TAC ATG TTC TGT GCC GGC TAC TCG GAT GGC   1159
   Ser Pro Asn Ile Thr Glu Tyr Met Phe Cys Ala Gly Tyr Ser Asp Gly
   320                 325                 330                 335
   AGC AAG GAC TCC TGC AAG GGG GAC AGT GGA GGC CCA CAT GCC ACC CAC   1207
   Ser Lys Asp Ser Cys Lys Gly Asp Ser Gly Gly Pro His Ala Thr His
                   340                 345                 350
   TAC CGG GGC ACG TGG TAC CTG ACG GGC ATC GTC AGC TGG GGC CAG GGC   1255
   Tyr Arg Gly Thr Trp Tyr Leu Thr Gly Ile Val Ser Trp Gly Gln Gly
               355                 360                 365
   TGC GCA ACC GTG GGC CAC TTT GGG GTG TAC ACC AGG GTC TCC CAG TAC   1303
   Cys Ala Thr Val Gly His Phe Gly Val Tyr Thr Arg Val Ser Gln Tyr
           370                 375                 380
   ATC GAG TGG CTG CAA AAG CTC ATG CGC TCA GAG CCA CGC CCA GGA GTC   1351
   Ile Glu Trp Leu Gln Lys Leu Met Arg Ser Glu Pro Arg Pro Gly Val
       385                 390                 395
   CTC CTG CGA GCC CCA TTT CCC TAG C CCAGCAGCCC TGGCCTGTGG           1396
   Leu Leu Arg Ala Pro Phe Pro
   400                 405
   AGAGAAAGCC AAGGCTGCGT CGAACTGTCC TGGCACCAAA TCCCATATAT TCTTCTGCAG 1456
   TTAATGGGGT AGAGGAGGGC ATGGGAGGGA GGGAGAGGTG GGGAGGGAGA CAGAGACAGA 1516
   AACAGAGAGA GACAGAGACA GAGAGAGACT GAGGGAGAGA CTCTGAGGAC ATGGAGAGAG 1576
   ACTCAAAGAG ACTCCAAGAT TCAAAGAGAC TAATAGAGAC ACAGAGATGG AATAGAAAAG 1636
   ATGAGAGGCA GAGGCAGACA GGCGCTGGAC AGAGGGGCAG GGGAGTGCCA AGGTTGTCCT 1696
   GGAGGCAGAC AGCCCAGCTG AGCCTCCTTA CCTCCCTTCA GCCAAGCCCC ACCTGCACGT 1756
   GATCTGCTGG CCCTCAGGCT GCTGCTCTGC CTTCATTGCT GGAGACAGTA GAGGCATGAA 1816
   CACACATGGA TGCACACACA CACACGCCAA TGCACACACA CAGAGATATG CACACACACG 1876
   GATGCACACA CAGATGGTCA CACAGAGATA CGCAAACACA CCGATGCACA CGCACATAGA 1936
   GATATGCACA CACAGATGCA CACACAGATA TACACATGGA TGCACGCACA TGCCAATGCA 1996
   CGCACACATC AGTGCACACG GATGCACAGA GATATGCACA CACCGATGTG CGCACACACA 2056
   GATATGCACA CACATGGATG AGCACACACA CACCAAGTGC GCACACACAC CGATGTACAC 2116
   ACACAGATGC ACACACAGAT GCACACACAC CGATGCTGAC TCCATGTGTG CTGTCCTCTG 2176
   AAGGCGGTTG TTTAGCTCTC ACTTTTCTGG TTCTTATCCA TTATCATCTT CACTTCAGAC 2236
   AATTCAGAAG CATCACCATG CATGGTGGCG AATGCCCCCA AACTCTCCCC CAAATGTATT 2296
   TCTCCCTTCG CTGGGTGCCG GGCTGCACAG ACTATTCCCC ACCTGCTTCC CAGCTTCACA 2356
   ATAAACGGCT GCGTCTCCTC CGCACACCTG TGGTGCCTGC CACCCAAAAA AAAAAAAAAA 2416
   AAAAAA 2422
(2) INFORMATION FOR SEQ ID NO:2:
   (i) SEQUENCE CHARACTERISTICS:
      (A) LENGTH: 444 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Val Ser Gln Ala Leu Arg Leu Leu Trp Leu Leu Leu Gly Leu Gln
   -38         -35                 -30                 -25
   Gly Cys Leu Ala Ala Val Phe Val Thr Gln Glu Glu Ala His Gly Val
           -20                 -15                 -10
   Leu His Arg Arg Arg Arg Ala Asn Ala Phe Leu Glu Glu Leu Arg Pro
       -5                   1                   5                  10
   Gly Ser Leu Glu Arg Glu Cys Lys Glu Glu Gln Cys Ser Phe Glu Glu
                   15                  20                  25
   Ala Arg Glu Ile Phe Lys Asp Ala Glu Arg Thr Lys Leu Phe Trp Ile
               30                  35                  40
   Ser Tyr Ser Asp Gly Asp Gln Cys Ala Ser Ser Pro Cys Gln Asn Gly
           45                  50                  55
   Gly Ser Cys Lys Asp Gln Leu Gln Ser Tyr Ile Cys Phe Cys Leu Pro
       60                  65                  70
   Ala Phe Glu Gly Arg Asn Cys Glu Thr His Lys Asp Asp Gln Leu Ile
   75                  80                  85                  90
   Cys Val Asn Glu Asn Gly Gly Cys Glu Gln Tyr Cys Ser Asp His Thr
                   95                  100                 105
   Gly Thr Lys Arg Ser Cys Arg Cys His Glu Gly Tyr Ser Leu Leu Ala
               110                 115                 120
   Asp Gly Val Ser Cys Thr Pro Thr Val Glu Tyr Pro Cys Gly Lys Ile
           125                 130                 135
   Pro Ile Leu Glu Lys Arg Asn Ala Ser Lys Pro Gln Gly Arg Ile Val
       140                 145                 150
   Gly Gly Lys Val Cys Pro Lys Gly Glu Cys Pro Trp Gln Val Leu Leu
   155                 160                 165                 170
   Leu Val Asn Gly Ala Gln Leu Cys Gly Gly Thr Leu Ile Asn Thr Ile
                   175                 180                 185
   Trp Val Val Ser Ala Ala His Cys Phe Asp Lys Ile Lys Asn Trp Arg
               190                 195                 200
   Asn Leu Ile Ala Val Leu Gly Glu His Asp Leu Ser Glu His Asp Gly
           205                 210                 215
   Asp Glu Gln Ser Arg Arg Val Ala Gln Val Ile Ile Pro Ser Thr Tyr
       220                 225                 230
   Val Pro Gly Thr Thr Asn His Asp Ile Ala Leu Leu Arg Leu His Gln
   235                 240                 245                 250
   Pro Val Val Leu Thr Asp His Val Val Pro Leu Cys Leu Pro Glu Arg
                   255                 260                 265
   Thr Phe Ser Glu Arg Thr Leu Ala Phe Val Arg Phe Ser Leu Val Ser
               270                 275                 280
   Gly Trp Gly Gln Leu Leu Asp Arg Gly Ala Thr Ala Leu Glu Leu Met
           285                 290                 295
   Val Leu Asn Val Pro Arg Leu Met Thr Gln Asp Cys Leu Gln Gln Ser
       300                 305                 310
   Arg Lys Val Gly Asp Ser Pro Asn Ile Thr Glu Tyr Met Phe Cys Ala
   315                 320                 325                 330
   Gly Tyr Ser Asp Gly Ser Lys Asp Ser Cys Lys Gly Asp Ser Gly Gly
                   335                 340                 345
   Pro His Ala Thr His Tyr Arg Gly Thr Trp Tyr Leu Thr Gly Ile Val
               350                 355                 360
   Ser Trp Gly Gln Gly Cys Ala Thr Val Gly His Phe Gly Val Tyr Thr
           365                 370                 375
   Arg Val Ser Gln Tyr Ile Glu Trp Leu Gln Lys Leu Met Arg Ser Glu
       380                 385                 390
   Pro Arg Pro Gly Val Leu Leu Arg Ala Pro Phe Pro
   395                 400                 405
(2) INFORMATION FOR SEQ ID NO:3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: N

   (xi) SEQUENCE DESCRIPTION: SEQ ID NO:3:
   TGGGCCTCCG GCGTCCCCCT T        21
(2) INFORMATION FOR SEQ ID NO:4:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: N

   (xi) SEQUENCE DESCRIPTION: SEQ ID NO:4:
   TCCCAGTCAC GACGT   15

## Description

### Field of the Invention

The present invention relates to proteins useful as anticoagulants. More specifically, the present invention relates to the use of modified forms of Factor VII that inhibit blood coagulation and tissue factor for inhibiting vascular restenosis as platelet deposition.

### Background Of The Invention

Blood coagulation is a process consisting of a complex interaction of various blood components, or factors, which eventually gives rise to a fibrin clot. Generally, the blood components which participate in what has been referred to as the coagulation "cascade" are proenzymes or zymogens, enzymatically inactive proteins which are converted to proteolytic enzymes by the action of an activator, itself an activated clotting factor. Coagulation factors that have undergone such a conversion and generally referred to as "active factors," and are designated by the addition of a lower case "a" suffix (e.g., Factor VIIa).

Activated Factor X ("Xa") is required to convert prothrombin to thrombin, which then converts fibrinogen to fibrin as a final stage in forming a fibrin clot. There are two systems, or pathways, that promote the activation of Factor X. The "intrinsic pathway" refers to those reactions that lead to thrombin formation through utilization of factors present only in plasma. A series of protease-mediated activations ultimately generates Factor IXa which, in conjunction with Factor VIIIa, cleaves Factor X into Xa. An identical proteolysis is effected by Factor VIIa and its cofactor, tissue factor, in the "extrinsic pathway" of blood coagulation. Tissue factor is a membrane bound protein and does not normally circulate in plasma. Upon vessel disruption, however, it can complex with Factor VIIa to catalyze Factor X activation or Factor IX activation in the presence of Ca⁺⁺ and phospholipid (Nemerson and Gentry, Biochem. 25:4020-4033 (1986)). While the relative importance of the two coagulation pathways in hemostasis is unclear, in recent years Factor VII and tissue factor have been found to play a pivotal role in the regulation of blood coagulation.

Factor VII is a trace plasma glycoprotein that circulates in blood as a single-chain zymogen. The zymogen is catalytically inactive (Williams et al., J. Biol. Chem. 264:7536-7543 (1989); Rao et al., Proc. Natl. Acad. Sci. USA. 85:6687-6691 (1988)). Single-chain Factor VII may be converted to two-chain Factor VIIa by Factor Xa, Factor XIIa, Factor IXa or thrombin in vitro. Factor Xa is believed to be the major physiological activator of Factor VII. Like several other plasma proteins involved in hemostasis, Factor VII is dependent on vitamin K for its activity, which is required for the γ-carboxylation of multiple glutamic acid residues that are clustered in the amino terminus of the protein. These γ-carboxylated glutamic acids are required for the metal-associated interaction of Factor VII with phospholipids.

The conversion of zymogen Factor VII into the activated two-chain molecule occurs by cleavage of an internal peptide bond located approximately in the middle of the molecule. In human Factor VII, the activation cleavage site is at Arg₁₅₂-Ile₁₅₃ (Hagen et al., Proc. Natl. Acad. Sci. USA 83: 2412-2416 (1986); Thim et al., Biochem. 27:7785-7793 (1988). Bovine factor VII is activated by cleavage at the analogous Arg₁₅₂-Ile₁₅₃ bond (Takeya et al., J. Biol. Chem. 263: 14868-14877, 1988). In the presence of tissue factor, phospholipids and calcium ions, the two-chain Factor VIIa rapidly activates Factor X or Factor IX by limited proteolysis.

International patent application WO 92/15686 discloses a modified Factor VII. The modifications render Factor VIIa substantially unable to activate plasma Factor X or IX. The use of the modified Factor VII in inhibiting vascular restenosis is not suggested.

It is often necessary to selectively block the coagulation cascade in a patient. Anticoagulants such as heparin, coumarin, derivatives of coumarin, indandione derivatives, or other agents may be used, for example, during kidney dialysis, or to treat deep vein thrombosis, disseminated intravascular coagulation (DIC), and a host of other medical disorders. For example, heparin treatment or extracorporeal treatment with citrate ion (U.S. Patent 4,500,309) may be used in dialysis to prevent coagulation during the course of treatment. Heparin is also used in preventing deep vein thrombosis in patients undergoing surgery.

Treatment with heparin and other anticoagulants may, however, have undesirable side effects. Available anticoagulants generally act throughout the body, rather than acting specifically at a clot site. Heparin, for example, may cause heavy bleeding. Furthermore, with a half-life of approximately 80 minutes, heparin is rapidly cleared from the blood, necessitating frequent administration. Because heparin acts as a cofactor for antithrombin III (AT III), and AT III is rapidly depleted in DIC treatment, it is often difficult to maintain the proper heparin dosage, necessitating continuous monitoring of AT III and heparin levels. Heparin is also ineffective if AT III depletion is extreme. Further, prolonged use of heparin may also increase platelet aggregation and reduce platelet count, and has been implicated in the development of osteoporosis. Indandione derivatives may also have toxic side effects.

In addition to the anticoagulants briefly described above, several naturally occurring proteins have been found to have anticoagulant activity. For example, Reutelingsperger (U.S. Patent No. 4,736,018) isolated anticoagulant proteins from bovine aorta and human umbilical vein arteries. Maki et al. (U.S.Patent No. 4,732,891) disclose human placenta-derived anticoagulant proteins. In addition, AT III has been proposed as a therapeutic anticoagulant (Schipper et al., Lancet 1 (8069): 854-856 (1978); Jordan, U.S. Patent No. 4,386,025; Bock et al., U.S. Patent No. 4,517,294).

Proliferation of smooth muscle cells (SMCs) in the vessel wall is an important event in the formation of vascular lesions in atherosclerosis, after vascular reconstruction or in response to other vascular injury. For example, treatment of atherosclerosis frequently includes the clearing of blocked vessels by angioplasty, endarterectomy or reduction atherectomy, or by bypass grafting, surgical procedures in which atherosclerotic plaques are compressed or removed through catheterization (angioplasty), stripped away from the arterial wall through an incision (endarterectomy) or bypassed with natural or synthetic grafts. These procedures remove the vascular endothelium, disturb the underlying intimal layer, and result in the death of medial SMCs. This injury is followed by medial SMC proliferation and migration into the intima, which characteristically occurs within the first few weeks and up to six months after injury and stops when the overlying endothelial layer is reestablished. In humans, these lesions are composed of about 20% cells and 80% extracellular matrix.

In about 30% or more of patients treated by angioplasty, endarterectomy or bypass grafts, thrombosis and/or SMC proliferation in the intima causes re-occlusion of the vessel and consequent failure of the reconstructive surgery. This closure of the vessel subsequent to surgery is known as restenosis.

There is still a need in the art for improved compositions having anticoagulant activity which can be administered at relatively low doses and do not produce the undesirable side effects associated with traditional anticoagulant compositions.

### Summary Of The Invention

The use of compositions which comprise modified Factor VII having anticoagulant properties is described for the inhibition of vascular restenosis or platelet deposition . The modified Factor VII compositions also inhibit tissue factor. The Factor VII sequence has at least one amino acid modification, where the modification is selected so as to substantially reduce the ability of activated Factor VII to catalyze the activation of plasma Factors X or IX, and thus is capable of inhibiting clotting activity. The Factor VII has an active site modified by at least one amino acid substitution, and in its modified form is capable of binding tissue factor. The modified Factor VII compositions are typically in substantially pure form.

The compositions are particularly useful for treating patients when formulated into pharmaceutical compositions, where they may be given to individuals suffering from a variety of disease states to treat coagulation-related conditions. Such modified Factor VII molecules, capable of binding tissue factor but having a substantially reduced ability to catalyze activation of other factors in the clotting cascade, may possess a longer plasma half-life and thus a correspondingly longer period of anticoagulative activity when compared to other anticoagulants. The compositions are used to inhibit vascular restenosis and platelet deposition. Thus, the present invention provides the use of Factor VII, having at least one modification in its catalytic center, which modification substantially inhibits the ability of the modified Factor VII to activate plasma Factor X or IX in the manufacture of a medicament for the inhibition of vascular restenosis or platelet deposition.

Typically, for administration to humans the pharmaceutical compositions will comprise modified human Factor VII protein and pharmaceutically-acceptable carriers and buffers.

In preferred embodiments of human and bovine Factor VII, the active site residue Ser₃₄₄ is modified, replaced with Gly, Met, Thr, or more preferably, Ala. Such substitution could be made separately or in combination with substitution(s) at other sites in the catalytic triad, which includes His₁₉₃ and Asp₂₄₂.

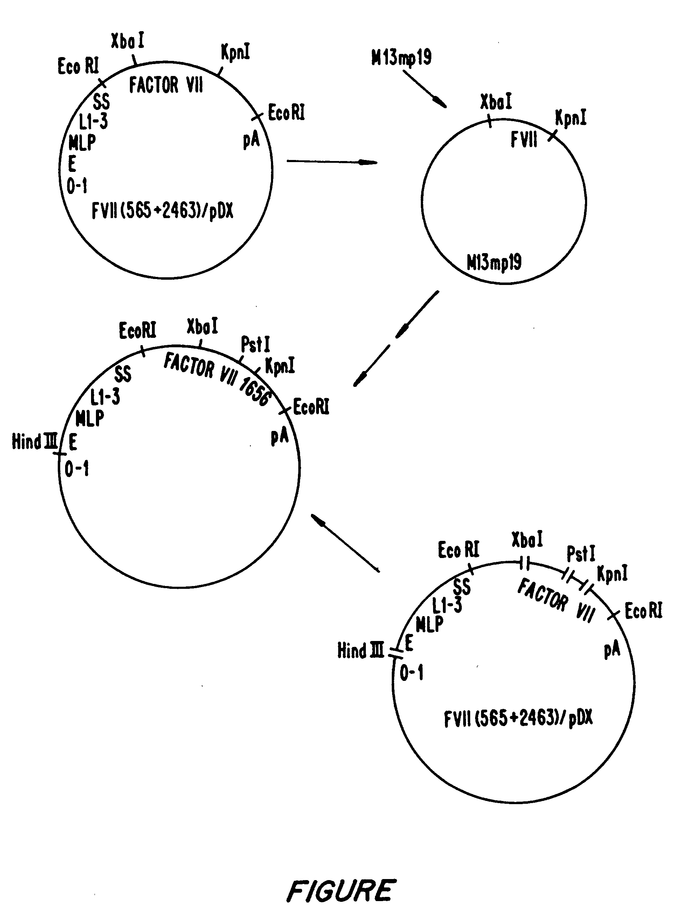

### Brief Description Of The Figure

The Figure illustrates the construction of an expression vector for a Ser₃₄₄→Ala modified Factor VII DNA sequence. Symbols used include 0-1, the 0-1 map unit sequence from adenovirus 5; E, the SV40 enhancer; MLP, the adenovirus 2 major late promotor; SS, a set of splice sites; and pA, the polyadenylation signal from SV40 in the late orientation.

### Description of The Specific Embodiments

Modified Factor VII can be in the form of the zymogen (i.e., a single-chain molecule) or can be cleaved at its activation site. Compositions of the modified Factor VII are suitable for administration to a variety of mammals, particularly humans, to inhibit the coagulation cascade. Modified Factor VII may be administered to a patient in conjunction with or in place of other anticoagulant compounds.

Factor VII plays an important role in the coagulation cascade, particularly that involving the extrinsic pathway. Present in the circulating plasma as an inactive single chain zymogen protein, once activated, Factor VIIa, in combination with tissue factor and calcium ions, activates Factor X to Xa and activates Factor IX to IXa, with the eventual formation of a fibrin clot.

The present invention provides the ability to inhibit this sequence of events in the coagulation cascade by preventing or otherwise inhibiting the activation of Factors X and IX by Factor VIIa. Factor VII proteins of the invention have a catalytic site which is modified to decrease the catalytic activity of Factor VIIa, while the molecule retains the ability to bind to tissue factor. The modified Factor VII molecules compete with native Factor VII and/or VIIa for binding to tissue factor. As a result, the activation of Factors X and IX is inhibited.

In one aspect of the present invention the catalytic activity of Factor VIIa is inhibited by chemical derivatization of the catalytic center, or triad. Derivatization may be accomplished by reacting Factor VII with an irreversible inhibitor such as an organophosphor compound, a sulfonyl fluoride, a peptide halomethyl ketone or an azapeptide, or by acylation, for example. Preferred peptide halomethyl ketones include PPACK (D-Phe-Pro-Arg chloromethyl ketone; see U.S. Patent No. 4,318,904), and DEGRck (dansyl-Glu-Gly-Arg chloromethyl ketone).

In another aspect, the catalytic activity of Factor VIIa can also be inhibited by substituting, inserting or deleting amino acids. In preferred embodiments amino acid substitutions are made in the amino acid sequence of the Factor VII catalytic triad, defined herein as the regions which contain the amino acids which contribute to the Factor VIIa catalytic site. The substitutions, insertions or deletions in the catalytic triad are generally at or adjacent to the amino acids which form the catalytic site. In the human and bovine Factor VII proteins, the amino acids which form a catalytic "triad" are Ser₃₄₄, Asp₂₄₂, and His₁₉₃ (subscript numbering indicating position in the sequence). The catalytic sites in Factor VII from other mammalian species may be determined using presently available techniques including, among others, protein isolation and amino acid sequence analysis. Catalytic sites may also be determined by aligning a sequence with the sequence of other serine proteases, particularly chymotrypsin, whose active site has been previously determined (Sigler et al., J. Mol. Biol., 35:143-164 (1968), and therefrom determining from said alignment the analogous active site residues.

The amino acid substitutions, insertions or deletions are made so as to prevent or otherwise inhibit activation by the Factor VIIa of Factors X and/or IX. The Factor VII so modified should, however, also retain the ability to compete with authentic Factor VII and/or Factor VIIa for binding to tissue factor in the coagulation cascade. Such competition may readily be determined by means of, e.g., a clotting assay as described herein, or a competition binding assay using, e.g., a cell line having cell-surface tissue factor, such as the human bladder carcinoma cell line J82 (Sakai et al. J. Biol. Chem. 264: 9980-9988 (1989).

The amino acids which form the catalytic site in Factor VII, such as Ser₃₄₄, Asp₂₄₂, and His₁₉₃ in human and bovine Factor VII, may either be substituted or deleted. Within the present invention, it is preferred to change only a single amino acid, thus minimizing the likelihood of increasing the antigenicity of the molecule or inhibiting its ability to bind tissue factor, however two or more amino acid changes (substitutions, additions or deletions) may be made and combinations of substitution(s), addition(s) and deletion(s) may also be made. In a preferred embodiment for human and bovine Factor VII, Ser₃₄₄ is preferably substituted with Ala, but Gly, Met, Thr or other amino acids can be substituted. It is preferred to replace Asp with Glu and to replace His with Lys or Arg. In general, substitutions are chosen to disrupt the tertiary protein structure as little as possible. The model of Dayhoff et al. (in Atlas of Protein Structure 1978, Nat'l Biomed. Res. Found., Washington, D.C.), may be used as a guide in selecting other amino acid substitutions. One may introduce residue alterations as described above in the catalytic site of appropriate Factor VII sequence of human, bovine or other species and test the resulting protein for a desired level of inhibition of catalytic activity and resulting anticoagulant activity as described herein. For the modified Factor VII the catalytic activity will be substantially inhibited, generally less than about 5% of the catalytic activity of wild-type Factor VII of the corresponding species, more preferably less than about 1%.

The proteins may be produced through the use of recombinant DNA techniques. In general, a cloned wild-type Factor VII DNA sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known. See U.S. Pat. No. 4,784,950, where the cloning and expression of recombinant human Factor VII is described. The bovine Factor VII sequence is described in Takeya et al., J. Biol. Chem. 263:14868-14872 (1988).

The amino acid sequence alterations may be accomplished by a variety of techniques. Modification of the DNA sequence may be by site-specific mutagenesis. Techniques for site-specific mutagenesis are well known in the art and are described by, for example, Zoller and Smith (DNA 3:479-488, 1984). Thus, using the nucleotide and amino acid sequences of Factor VII, one may introduce the alteration(s) of choice.

The modified Factor VII includes those proteins that have the amino-terminal portion (gla domain) substituted with a gla domain of one of the vitamin K-dependent plasma proteins Factor IX, Factor X, prothrombin, protein C, protein S or protein Z. The gla domains of the vitamin K-dependent plasma proteins are characterized by the presence of gamma-carboxy glutamic acid residues and are generally from about 30 to about 40 amino acids in length with C-termini corresponding to the positions of exon-intron boundaries in the respective genes. Methods for producing Factor VII with a heterologous gla domain are disclosed in U.S. Patent No. 4,784,950.

DNA sequences for use within the present invention will typically encode a pre-pro peptide at the amino-terminus of the Factor VII protein to obtain proper post-translational processing (e.g. gamma-carboxylation of glutamic acid residues) and secretion from the host cell. The pre-pro peptide may be that of Factor VII or another vitamin Kdependent plasma protein, such as Factor IX, Factor X, prothrombin, protein C or protein S. As will be appreciated by those skilled in the art, additional modifications can be made in the amino acid sequence of the modified Factor VII where those modifications do not significantly impair the ability of the protein to act as an anticoagulant. For example, the Factor VII modified in the catalytic triad can also be modified in the activation cleavage site to inhibit the conversion of zymogen Factor VII into its activated two-chain form, as generally described in U.S. Patent 5,288,629.

Expression vectors will comprise a promoter capable of directing the transcription of a cloned gene or cDNA. Preferred promoters for use in cultured mammalian cells include viral promoters and cellular promoters. Viral promoters include the SV40 promoter (Subramani et al., Mol. Cell. Biol. 1:854-864, 1981) and the CMV promoter (Boshart et al., Cell 41:521-530, 1985). A particularly preferred viral promoter is the major late promoter from adenovirus 2 (Kaufman and Sharp, Mol. Cell. Biol. 2:1304-1319, 1982). Cellular promoters include the mouse kappa gene promoter (Bergman et al., Proc. Natl. Acad. Sci. USA 81:7041-7045, 1983) and the mouse V_{H} promoter (Loh et al., Cell 33:85-93, 1983). A particularly preferred cellular promoter is the mouse metallothionein-I promoter (Palmiter et al., Science 222:809-814, 1983). Expression vectors may also contain a set of RNA splice sites located downstream from the promoter and upstream from the insertion site for the Factor VII sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the human growth hormone gene terminator (DeNoto et al. Nuc. Acids Res. 9:3719-3730, 1981) or the polyadenylation signal from the human Factor VII gene or the bovine Factor VII gene. The expression vectors may also include a noncoding viral leader sequence, such as the adenovirus 2 tripartite leader, located between the promoter and the RNA splice sites; and enhancer sequences, such as the SV40 enhancer.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers are reviewed by Thilly (Mammalian Cell Technology, Butterworth Publishers, Stoneham, MA. The choice of selectable markers is well within the level of ordinary skill in the art.

Selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing a dicistronic message. Constructs of this type are known in the art (for example, Levinson and Simonsen, U.S. Patent 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells and the expression of the modified Factor VII gene. Media generally include a carbon source, a nitrogen source, essential amino acids, essential sugars, vitamins, salts, phospholipids, protein and growth factors. For production of gamma-carboxylated modified Factor VII, the medium will contain vitamin K, preferably at a concentration of about 0.1 µg/ml to about 5 µg/ml. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby increasing expression levels. Clones of stably transfected cells are then screened for expression of modified Factor VII.

Preferred mammalian cell lines include the COS-1 (ATCC CRL 1650), baby hamster kidney (BHK) and 293 (ATCC CRL 1573; Graham et al., J. Gen. Virol. 36:59-72, 1977) cell lines. A preferred BHK cell line is the tk⁻ ts13 BHK cell line (Waechter and Baserga, Proc. Natl. Acad. Sci. USA 79:1106-1110, 1982, incorporated herein by reference), hereinafter referred to as BHK 570 cells. The BHK 570 cell line has been deposited with the American Type Culture Collection, 12301 Parklawn Dr., Rockville, MD 20852, under ATCC accession number CRL 10314. A tk⁻ ts13 BHK cell line is also available from the ATCC under accession number CRL 1632. In addition, a number of other cell lines may be used, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77:4216-4220, 1980).

Modified Factor VII produced may be purified by affinity chromatography on an anti-Factor VII antibody column. The use of calcium-dependent monoclonal antibodies, as described by Wakabayashi et al., J. Biol. Chem. 261:11097-11108, (1986) and Thim et al., Biochem. 27: 7785-7793, (1988), is particularly preferred. Additional purification may be achieved by conventional chemical purification means, such as high performance liquid chromatography. Other methods of purification, including barium citrate precipitation, are known in the art, and may be applied to the purification of the modified Factor VII described herein (see, generally, Scopes, R., Protein Purification, Springer-Verlag, N.Y., 1982). Substantially pure modified Factor VII of at least about 90 to 95% homogeneity is preferred, and 98 to 99% or more homogeneity most preferred, for pharmaceutical uses. Once purified, partially or to homogeneity as desired, the modified Factor VII may then be used therapeutically.

Within one embodiment the modified Factor VII is cleaved at its activation site to convert it to its two-chain form. Activation may be carried out according to procedures known in the art, such as those disclosed by Osterud, et al., Biochemistry 11:2853-2857 (1972); Thomas, U.S. Patent No. 4,456,591; Hedner and Kisiel, J. Clin. Invest. 71:1836-1841 (1983); or Kisiel and Fujikawa, Behring Inst. Mitt. 73:29-42 (1983). The resulting modified "Factor VIIa" is then formulated and administered as described below.

The modified Factor VII or VIIa molecules and pharmaceutical compositions thereof are particularly useful for administration to humans to inhibit vascular restenosis or platelet deposition.

The modified Factor VII or VIIa molecules may be particularly useful in the treatment of restenosis due to acute vascular injury. Acute vascular injuries are those which occur rapidly (i.e. over days to months), in contrast to chronic vascular injuries (e.g. atherosclerosis) which develop over a lifetime. Acute vascular injuries often result from surgical procedures such as vascular reconstruction, wherein the techniques of angioplasty, endarterectomy, atherectomy, vascular graft emplacement or the like are employed. Hyperplasia may also occur as a delayed response in response to, e.g., graft emplacement or organ transplantation. Since modified Factor VII is more selective than heparin, generally binding only tissue factor which has been exposed at sites of injury, and because modified Factor VII does not destroy other coagulation proteins, it will be more effective and less likely to cause bleeding complications that heparin when used prophylactically for the prevention of deep vein thrombosis. The dose of modified Factor VII for prevention of deep vein thrombosis is in the range of about 50 µg to 25 mg/day, preferably 1 to 10 mg/day for a 70 kg patient, and administration should begin at least about 6 hours prior to surgery and continue at least until the patient becomes ambulatory. The dose of modified Factor VII or VIIa in the treatment for restenosis will vary with each patient but will generally be in the range of those suggested above.

Recent advances in the treatment of coronary vascular disease include the use of mechanical interventions to either remove or displace offending plaque material in order to re-establish adequate blood flow through the coronary arteries. Despite the use of multiple forms of mechanical interventions, including balloon angioplasty, reduction atherectomy, placement of vascular stents, laser therapy, or rotoblator, the effectiveness of these techniques remains limited by an approximately 40% restenosis rate within 6 months after treatment.

Restenosis is thought to result from a complex interaction of biological processes including platelet deposition and thrombus formation, release of chemotactic and mitogenic factors, and the migration and proliferation of vascular smooth muscle cells into the intima of the dilated arterial segment.

The inhibition of platelet accumulation at sites of mechanical injury can limit the rate of restenosis in human subjects. Therapeutic use of a monoclonal antibody to platelet GpIIb/IIIa is able to limit the level of restenosis in human subjects (Califf et al., N. Engl. J. Med., 330:956-961 (1994)). The antibody is able to bind to the GpIIb/IIIa receptor on the surfaces of platelets and thereby inhibit platelet accumulation. This data suggests that inhibition of platelet accumulation at the site of mechanical injury in human coronary arteries is beneficial for the ultimate healing response that occurs. While platelet accumulation occurs at sites of acute vascular injuries, the generation of thrombin at these sites may be responsible for the activation of the platelets- and their subsequent accumulation.

As shown in the examples that follow, the modified Factor VIIa of the present invention is able to bind to cell-surface tissue factor. For example, DEGR-Factor VIIa binds cell-surface tissue factor with an equivalent or higher affinity than wild-type Factor VIIa. DEGR-Factor VIIa, however, has no enzymatic activity, yet it binds to tissue factor and acts as a competitive antagonist for wild-type Factor VIIa, thereby inhibiting the subsequent steps in the extrinsic pathway of coagulation leading to the generation of thrombin.

Modified Factor VIIa molecules maintain tissue factor inhibit platelet accumulation at the site of vascular injury by blocking the production of thrombin and the subsequent deposition of fibrin.

Due to the ability of DEGR-Factor VIIa to block thrombin generation and limit platelet deposition at sites of acute vascular injury, modified Factor VIIa molecules maintain tissue factor binding activity but lack Factor VIIa enzymatic activity can be used to inhibit vascular restenosis.

In established deep vein thrombosis and/or pulmonary embolism, the dose of modified Factor VII ranges from about 50 µg to 25 mg as a loading dose followed by maintenance doses ranging from about 500 µg to 10 mg/day, depending on the weight of the patient and the severity of the condition. Because of the lower likelihood of bleeding complications from modified Factor VII infusions, modified Factor VII can replace or lower the dose of heparin during or after surgery in conjunction with thrombectomies or embolectomies.

The modified Factor VII compositions will also have substantial utility in the prevention of cardiogenic emboli and in the treatment of thrombotic strokes. Because of its low potential for causing bleeding complications and its selectivity, modified Factor VII can be given to stroke victims and may prevent the extension of the occluding arterial thrombus. The amount of modified Factor VII administered will vary with each patient depending on the nature and severity of the stroke, but doses will generally be in the range of those suggested below.

Pharmaceutical compositions of modified Factor VII will be a useful treatment in acute myocardial infarction. because of the ability of modified Factor VII to inhibit in vivo coagulation. Modified Factor VII can be given with tissue plasminogen activator or streptokinase during the acute phases of the myocardial infarction. In acute myocardial infarction, the patient is given a loading dose of at least about 1 to 25 mg of modified Factor VII, followed by maintenance doses of about 500 µg to about 10 mg/day.

The modified Factor VII is useful in the treatment of disseminated intravascular coagulation (DIC). Patients with DIC characteristically have widespread microcirculatory thrombi and often severe bleeding problems which result from consumption of essential clotting factors. Because of its selectivity, modified Factor VII will not aggravate the bleeding problems associated with DIC, as do conventional anticoagulants, but will retard or inhibit the formation of additional microvascular fibrin deposits.

The pharmaceutical compositions are intended for parenteral, topical or local administration for prophylactic and/or therapeutic treatment. Preferably, the pharmaceutical compositions are administered parenterally, i.e., intravenously, subcutaneously, or intramuscularly. Compositions for parenteral administration comprise a solution of the modified Factor VII molecules dissolved in an acceptable carrier, preferably an aqueous carrier. A variety of aqueous carriers may be used, e.g., water, buffered water, 0.4% saline, 0.3% glycine and the like. The modified Factor VII molecules can also be formulated into liposome preparations for delivery or targeting to sites of injury. Liposome preparations are generally described in, e.g., U.S. 4,837,028, U.S. 4,501,728, and U.S. 4,975,282. The compositions may be sterilized by conventional, well known sterilization techniques. The resulting aqueous solutions may be packaged for use or filtered under aseptic conditions and lyophilized, the lyophilized preparation being combined with- a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. The concentration of modified Factor VII in these formulations can vary widely, i.e., from less than about 0.5%, usually at or at least about 1% to as much as 15 or 20% by weight and will be selected primarily by fluid volumes, viscosities, etc., in accordance with the particular mode of administration selected.

Thus, a typical pharmaceutical composition for intravenous infusion could be made up to contain 250 ml of sterile Ringer's solution, and 10 mg of modified Factor VII. Actual methods for preparing parenterally administrable compounds will be known or apparent to those skilled in the art and are described in more detail in for example, Remington's Pharmaceutical Science, 16th ed., Mack Publishing Company, Easton, PA (1982).

The compositions containing the modified Factor VII molecules can be administered for prophylactic and/or therapeutic treatments. In therapeutic applications, compositions are administered to a patient already suffering from a disease, as described above, in an amount sufficient to cure or at least partially arrest the disease and its complications. An amount adequate to accomplish this is defined as "therapeutically effective dose." Amounts effective for this use will depend on the severity of the disease or injury and the weight and general state of the patient, but generally range from about 0.05 mg to about 25 mg of modified Factor VII per day for a 70 kg patient, with dosages of from about 0.5 mg to about 10 mg of modified Factor VII per day being more commonly used. It must be kept in mind that the materials may generally be employed in serious disease or injury states, that is, life-threatening or potentially life threatening situations. In such cases, in view of the minimization of extraneous substances and general lack of immunogenicity of modified human Factor VII in humans, it is possible and may be felt desirable by the treating physician to administer substantial excesses of these modified Factor VII compositions.

In prophylactic applications, compositions containing the modified Factor VII are administered to a patient susceptible to or otherwise at risk of a disease state or injury- to enhance the patient's own anticoagulative capabilities. Such an amount is defined to be a "prophylactically effective dose." In this use, the precise amounts again depend on the patient's state of health and weight, but generally range from about 0.1 mg to about 25 mg per 70 kilogram patient, more commonly from about 0.5 mg to about 10 mg per 70 kg of body weight.

Single or multiple administrations of the compositions can be carried out with dose levels and pattern being selected by the treating physician. For ambulatory patients requiring daily maintenance levels, the modified Factor VII may be administered by continuous infusion using a portable pump system, for example. Local delivery of the modified Factor VII or VIIa may be carried out, for example, by way of perfusion, double balloon catheters, stent or other well established methods. In any event, the pharmaceutical formulations should provide a quantity of modified Factor VII sufficient to effectively treat the patient.

The following examples are offered by way of illustration, not by way of limitation.

### EXAMPLE I

### Expression of Ser₃₄₄→Ala₃₄₄ Factor VII

To generate the Ser₃₄₄→Ala Factor VII active site mutant, plasmid FVII(565+2463)/ pDX (U.S. Patent No. 4,784,950; deposited with the American Type Culture Collection under accession number 40205) was digested with Xba I and Kpn I, and the resulting 0.6 kb fragment, comprising the coding region for serine 344, was recovered. This fragment was cloned into Xba I, Kpn I-digested M13mp19 as shown in the Figure. This manipulation and subsequent steps described below were generally performed according to standard protocols (as described, for example, by Maniatis et al., Molecular Cloning A Laboratory Manual, Cold Spring Harbor Laboratory Press, Cold Spring Harbor, N.Y. (1982).

Mutagenesis was carried out on the M13 template according to the methods of Zoller and Smith, supra, using the mutagenic oligonucleotide ZC1656 (5' TGG GCC TCC GGC GTC CCC CTT 3') and the "universal" second primer ZC87 (5' TCC CAG TCA CGA CGT 3'). Reaction products were screened using kinased ZC1656. Positive plaques were picked, and template DNA was prepared and sequenced from the Pst I site at 1077 to the Kpn I site at 1213. Sequence analysis confirmed the presence of the desired mutation. The mutant clone was designated 1656.

An expression vector was then constructed using the 1656 clone. The mutagenized sequence was isolated from the M13 vector as a ~0.14 kb Pst I-Kpn I fragment. This fragment was ligated to the 1.7 kb Hind III-Xba I fragment from FVII(565+2463)/pDX, the 0.5 kb Xba I-Pst I fragment from FVII(565+2463)/pDX, and the 4.3 kb Kpn I-Hind III fragment from FVII(565+2463)/pDX, as shown in the Figure. The presence of the desired mutant sequence was confirmed by digesting mutant and wild-type clones with Pst I, and a mutant Factor VII insert in M13 with Kpn I and Xba I, preparing Southern blots of the digested DNA, and probing the blots with radiolabeled ZC1656.

The baby hamster kidney cell line BHK 570 (deposited with the American Type Culture Collection under accession number 10314) was transfected with two isolates (designated #544 and #545) of the 1656 expression vector. The cells were prepared by diluting a confluent 10 cm plate of BHK 570 cells 1:10 into five 10 cm plates in non-selective medium (Dulbecco's modified Eagle's medium [DMEM] containing 10% fetal bovine serum and 1% PSN antibiotic mix [GIBCO Life Technologies, Gaithersburg, MD]). After 24 hours, when the cells had reached 20-30% confluency, they were co-transfected with one isolate of the expression vector encoding the 1656 mutation, plasmid p486 (comprising the Adenovirus 5 ori, SV40 enhancer, Adenovirus 2 major late promotor, Adenovirus 2 tripartite leader, 5' and 3' splice sites, the DHFR^{r} cDNA and SV40 polyadenylation signal in pML-1 (Lusky and Botchan, Nature 293: 79-81, (1981)) and 10 µg of carrier DNA (sonicated salmon sperm DNA) as shown in Table 1. The DNA was added to a 15 ml tube, then 0.5 ml of 2X Hepes (25 g Hepes, 40 g NaCl, 1.8 g KCl, 0.75 g Na₂HPO₄·2H₂O, 5 g dextrose diluted to 2.5 1 with distilled water and pH adjusted to pH 6.95-7.0) was added and the tubes were mixed. The DNA in each tube was precipitated by the addition of 0.5 ml of 0.25 M CaCl₂ while air was bubbled through the DNA/Hepes solution with a pasteur pipet. The tubes were then vortexed, incubated at room temperature for 15 minutes, and vortexed again. The DNA mixtures were then added dropwise onto the plates of cells with a pipette. The plates were swirled and incubated at 37°C for 4-6 hours. After incubation, 2 ml of 20% glycerol diluted in Tris-saline (0.375 g KCl, 0.71 g Na₂HPO₄, 8.1 g NaCl, 3.0 g Tris-HCl, 0.5 g sucrose, diluted in a total of 1 liter and pH adjusted to pH 7.9) was then added to each plate. The plates were swirled and left at room temperature for two minutes. The medium was then removed from the plates and replaced with 2 ml of Tris-saline. The plates were left at room temperature for 2 minutes, then the Tris-saline was removed and replaced with 10 ml of non-selective medium. The plates were incubated at 37°C for two days.

**Table 1**

| | Transfection* | | | |
|---|---|---|---|---|
| | 544 | 545 | 544 Control | 545 Control |
| Plasmid Name | | | | |
| Clone 544 | 15 µl | --- | 15 µl | --- |
| Clone 545 | --- | 30 µl | --- | 30 µl |
| p486 | 1.5 µl | 1.5 µl | --- | --- |
| Carrier DNA | 1.6 µl | 1.6 µl | 1.6 µl | 1.6 µl |

| | | | | |
|---|---|---|---|---|
| * DNA concentrations used were: clone 544, 0.7 µg/µl; clone 545, 0.3 µg/µl; p486, 1.49 µg/µl. | | | | |

After the two day incubation, the cells were diluted in selection medium (DMEM containing 10% dialyzed fetal bovine serum, 1% PSN antibiotic mix and 150 nM methotrexate) and plated at dilutions of 1:100, 1:250 and 1:500 in maxi plates. The plates were incubated at 37°C for one week. After one week, the medium was changed and replaced with selection medium, and the plates were checked for colony formation.

Eight days later, after colony formation, twelve colonies were randomly chosen from the 1:500 dilution plates of the #544 and #545 transfection plates. Each clone was plated into one well of a 6-well plate and grown in selection medium. After seven days, the plates were confluent, and the clones were each split into 10 cm plates in selection medium.

The clones described above and control cells transfected to express wild-type factor VII were metabolically labeled with ³⁵S-Methionine-Cysteine Protein Labeling Mix (NEN DuPont Biotechnology Systems, Wilmington, DE). The clones were grown and prepared for a pulse label experiment in selective medium. The cells were rinsed with phosphate buffered saline (Sigma, St. Louis, MO) and pulsed for four hours in 20 µCi/ml ³⁵S-Cys-³⁵S-Met. After four hours, supernatants and cells were harvested. The cells were lysed essentially as described by Lenk and Penman (Cell 16: 289-302, (1979)) and 400 µl of each lysate and precleared with 50 µl of staph A (Sigma, St. Louis, MO).

Samples from the metabolically labeled cells were radioimmunoprecipitated (RIP) by first incubating the samples with 6 µl of anti-Factor VII polyclonal antisera for four hours. Sixty microliters of washed staphylococcal protein A was added to each sample, and the samples were rocked for 1.5 hours at 4°C. The samples were centrifuged, and the supernatant was removed. The pellets were washed twice in 0.7 M RIPA buffer (10 mM Tris, pH 7.4, 1% deoxycholic acid [Calbiochem Corp., La Jolla, CA], 1% Triton X-100, 0.1% SDS, 5 mM EDTA, 0.7 M NaCl) and once in 0.15 M RIPA buffer (10 mM Tris, pH 7.4, 1% deoxycholic acid [Calbiochem Corp., La Jolla, CA], 1% Triton X-100, 0.1 SDS, 5 mM EDTA, 0.15 M NaCl). One hundred microliters of 1x SDS dye (50 mM Tris-HCl, pH 6.8, 100 mM dithiothreitol, 2% SDS, 0.1% bromphenol blue, 10% glycerol) was added to each sample, and the samples were boiled for 5 minutes followed by centrifugation to remove the protein A. Fifty microliters of each sample was run on a 10% polyacrylamide gel. Results showed that 9 of 10 clones secreted modified Factor VII.

### EXAMPLE II

### ANTICOAGULANT ACTIVITY OF MODIFIED FACTOR VII

The ability of the modified Factor VII protein to inhibit clotting was measured in a one-stage clotting assay using wild-type Factor VII as a control. Recombinant proteins were prepared essentially as described above from cells cultured in media containing 5µg/ml vitamin K. Varying amounts of the modified Factor VII (from clone 544) or recombinant wild-type Factor VII were diluted in 50 mM Tris pH 7.5, 0.1% BSA to 100 µl. The mixtures were incubated with 100 µl of Factor VII-deficient plasma (George King Bio-Medical Inc., Overland Park, KS) and 200 µl of thromboplastin C (Dade, Miami, FL; contains rabbit brain thromboplastin and 11.8 mM Ca⁺⁺). The clotting assay was performed in an automatic coagulation timer (MLA Electra 800, Medical Laboratory Automation Inc., Pleasantville, NY), and clotting times were converted to units of Factor VII activity using a standard curve constructed with 1:5 to 1:640 dilutions of normal pooled human plasma (assumed to contain one unit per ml Factor VII activity; prepared by pooling citrated serum from healthy donors). Using this assay the preparations of modified Factor VII exhibited no detectable coagulant activity. Table 2 shows results of the assay in terms of clotting times for control (untransfected) BHK cell-conditioned media (+/- vitamin K), wild-type Factor VII and two isolates of cells expressing the modified Factor VII. Factor VII activity is seen as a reduction in clotting time over control samples.

**Table 2**

| Sample | Dilution | Clotting Time (sec.) |
|---|---|---|
| Control +K | 1:5 | 33.1 |
| | 1:10 | 33.4 |
| | | |
| Control -K | 1:5 | 34.3 |
| | 1:10 | 33.2 |
| | | |
| Wild-type | 1:20 | 19.0 |
| Factor VII | 1:40 | 21.5 |
| | 1:80 | 23.3 |
| | | |
| Modified Factor VII (#6) | 1:1 | 33.5 |
| | | |
| Modified Factor VII (#10) | 1:1 | 32.5 |

To determine the effect of the modified Factor VII on plasma factor substrates, preparations of modified Factor VII and recombinant wild-type or native Factor VII are incubated with either Factor X or Factor IX and the activation thereof monitored by clotting assays or polyacrylamide gel electrophoresis.

### EXAMPLE III

### Ability of Modified Factor VII to Bind Tissue Factor

The ability of the modified Factor VII to compete with wild-type Factor VII for tissue factor and inhibit its clotting activity was assessed in a one-step clotting assay in the presence of a limiting amount of tissue factor (thromboplastin).

Clotting times were determined in a one-step assay similar to that described in Example II. A limited amount of tissue factor, a constant amount of wild type Factor VII, and increasing amounts of variant Factor VII were used in the mixing experiments. An inhibition of Factor VII/VIIa procoagulant activity would be seen as an increase in clotting time in assays containing increasing amounts of variant Factor VII.

The amount of Factor VII activity in the test samples was calculated as a percentage of a standard curve that measured Factor VII activity in normal pooled plasma. The standard curve for Factor VII activity was generated using serial dilutions of normal pooled plasma in phosphate buffered solution (PBS) that ranged from 1:5 to 1:640. For this purpose it was assumed that normal plasma contains approximately 500 ng/ml of Factor VII and this was considered to be one unit of activity. A mixture of 100 µl Factor VII-deficient plasma, 100 µl plasma dilution and 200 µl of thromboplastin-C (Dade, Miami, FL.) was used to measure clotting time on a MLA Electra 800 automatic timer. To establish the standard curve, the results were graphed as percentage of activity (1:5 = 100% activity) versus clotting time in seconds.

The assay required that the medium containing the wild type and variant Factor VII be composed of less than one percent serum. The dilutions were made in PBS so that clotting times would fall along the standard curve. A minimum dilution of 1:2 was typical. The final volume was 100 µl. Two different human Factor VII Ser₃₄₄ → Ala variants, designated clones "#10" and "#6" were tested in the experiments. The results, set forth in the Table below, show that as the amount of Factor VII variant increased, the percent of Factor VIIa activity decreased.

**Table 3:**

| Results of mixing assay with Ser344 → Ala Variants (B4A1 (wild type) medium was used as 100% activity at 10 µl/reaction) | | | | |
|---|---|---|---|---|
| Ser344 → Ala Clone No. | Variant medium amount | B4A1 medium amount | BHK Control* | Percent FVIIa Activity |
| #10 | 10 µl | 10 µl | 0 | 70 |
| #10 | 20 µl | 10 µl | 0 | 51 |
| #10 | 30 µl | 10 µl | 0 | 43 |
| #10 | 40 µl | 10 µl | 0 | 34 |
| #10 | 50 µl | 10 µl | 0 | 28 |
| #10 (-K)^{§} | 20 µl | 10 µl | 0 | 78 |
| #6 | 10 µl | 10 µl | 0 | 74 |
| #6 | 20 µl | 10 µl | 0 | 56 |
| #6 | 30 µl | 10 µl | 0 | 46 |
| #6 | 40 µl | 10 µl | 0 | 41 |
| #6 | 50 µl | 10 µl | 0 | 32 |
| #6 (-K) | 20 µl | 10 µl | 0 | 85 |
| | | | | |
| BHK control | 0 | 10 µl | 20 µl | 91 |
| BHK control (-K) | 0 | 10 µl | 20 µl | 107 |

| | | | | |
|---|---|---|---|---|
| * Untransfected conditioned medium | | | | |
| ^{§} For expression of the Factor VII variant, cells were grown in the presence of vitamin K, except where noted "(-K)". | | | | |

These experiments showed that variants of Factor VII having a Ser₃₄₄ → Ala substitution competed with native Factor VII in a dose dependent fashion and inhibited the procoagulant activity of native Factor VII/VIIa. It can thus be concluded that Ser₃₄₄ → Ala variant human Factor VII competes with native human Factor VIIa and consequently inhibits activation of Factor X and/or IX in human plasma.

### EXAMPLE IV

### Reaction of Factor VII with PPACK

Recombinant Factor VII was produced in transfected baby hamster kidney cells. The protein was purified and activated as disclosed by Thim et al. (Biochemistry 27: 7785-7793, 1988), Brinkous et al. (Proc. Natl.Acad. Sci. USA 86: 1382-1386, 1989) and Bjoern and Thim (Res. Discl. No. 269, 564, 1986), which are incorporated herein by reference. The cell culture medium was recovered, filtered and diluted to reduce sale concentration. The diluted medium was then fractionated by anion exchange chromatography using an elution buffer containing CaCl₂. The Factor VII fraction was recovered and further purified by immunochromatography using a calcium-dependent anti-Factor VII monoclonal antibody. Additional purification was carried out using two anion exchange chromatography steps wherein Factor VII was eluted using CaCl₂ and NaCl, respectively. Factor Vlla was recovered in the final eluate.

Recombinant Factor Vlla (1 µM) in 50 mM Tris-HCl, 100 mM NaCl, 5 mM CaCl₂, ph 7.4 was incubated with 20 µM PPack (D-Phenylalanyl-Prolyl-Arginyl Chloromethyl Ketone; Calbiochem, La Jolla, CA) for 5, 20 and 60 minutes. Buffer containing the chromogenic substrate S2288 (H-D-Isoleucine-L-Prolyl-L-Arginine p-nitroanilide; Kabi Vitrum AB, Molndal, Sweden) was then added to obtain a 2.5 fold dilution and a final concentration of 0.3 mM S2288. The generation of p-nitroaniline was measured and compared to results using untreated Factor Vlla as a control. The results indicated that Factor Vlla is fully inactivated after about 60 minutes under these reaction conditions.

### EXAMPLE V

### Generation of DEGR-Factor VIIa

Recombinant human Factor VIIa was prepared as described in Example IV. Recombinant human Factor VIIa, in 10 mM glycine buffer, pH 8.0, 10 mM CaCl₂, 50 mM NaCl, was diluted to a concentration of 1.5 mg/ml. A 10-fold molar excess of Dansyl-L-Glu-Gly-Arg-Chloromethyl Ketone, DEGRck, (Calbiochem, La Jolla, CA 92037) which had been dissolved with distilled H₂O was added to the Factor VIIa. After a 2 hr incubation at 37°C, a second 10-fold molar excess of DEGRck was added to the mixture and incubated for an additional 2 hr at 37°C. A third 10-fold molar excess of DEGRck was added to the Factor VIIa and incubated for approximately 16 hours at 4°C. The DEGR-Factor VIIa sample was then extensively dialyzed at 4°C against Tris buffered saline (0.05 M Tris-HCl, 0.1 M NaCl, pH 7.5) to remove any free DEGRck.

The final DEGR-Factor VIIa mixture was tested for the presence of free DEGRck in a Factor Xa chromogenic substrate assay. The DEGR-Factor VIIa mixture was added to purified human Factor Xa along with the chromogenic substrate S-2222. This substrate is cleaved specifically by Factor Xa and not by Factor VIIa. Unbound DEGRck in the mixture is able to bind to the Factor Xa and there by inhibit the chromogenic activity of the Factor Xa. Spiking free DEGR-ck into a Factor Xa mixture generated a standard curve to measure the level of free DEGRck in solution versus the inhibition of Factor Xa chromogenic activity. Analysis of the DEGR-Factor VIIa mixture showed that the ratio of free DEGRck:DEGR-Factor VIIa was less than 0.5% following extensive dialysis, thereby ensuring that the inhibition observed by DEGR-Factor VIIa in the various assay systems described below was not due to the presence of free DEGRck.

### Example VI

### Factor Xa generation on rat smooth muscle cells

Vascular smooth muscle cells were analyzed for the presence of cell-surface tissue factor by measuring the ability of the cells to stimulate the conversion of Factor X to Factor Xa using a chromogenic substrate that is specific for Factor Xa.

Rat vascular smooth muscle cells (Clowes et al., J. Clin. Invest. 93:644-651 (1994)) were plated into 96-well culture dishes (American Scientific Products, Chicago, Il.) at 8,000 cells per well in growth media (Table 4).

**Table 4**

| |
|---|
| 500 ml Dulbecco's Modified Eagle's Medium (DMEM) (GIBCO-BRL, Gaithersburg, MD.) |
| 10% fetal calf serum (Hyclone, Logan, UT.) |
| 1mM sodium pyruvate (Irvine, Santa Ana, CA.) |
| 0.29 mg/ml L-glutamine (Hazelton, Lenexa,KS.) |
| 1x PSN; (100X is 5 mg/ml penicillin, 5 mg/ml streptomycin, 10 mg/ml neomycin) (GIBCO-BRL, Gaithersburg, MD.) |

After a 48 hour incubation at 37°C the medium was changed to serum free medium (Table 5).

**Table 5**

| |
|---|
| 250 ml Dulbecco's Modified Eagle's Medium (DMEM) |
| 250 ml Ham's F-12 Medium (Fred Hutchinson Cancer Research Center, Seattle, WA) |
| 1mM sodium pyruvate |
| 0.29 mg/ml L-glutamine |
| 20 µM transferrin (JRH, Lenexa, KS.) |
| 5 µM insulin (GIBCO-BRL) |
| 16 ng selenium (Aldrich, Milwaukee, WI.) |
| 1 mg/ml bovine serum albumin (Sigma, St. Louis, MO) |

The cells were incubated 72 hours at 37°C. After incubation, either PDGF-BB (10 ng/ml) or 10% fetal calf serum was added to the cells to stimulate tissue factor expression (Taubman et al., J. Clin. Invest. 91:547-552, 1993). A parallel set of cells received neither PDGF nor serum to monitor for intrinsic activity of unstimulated cells. After a 6 hour incubation, recombinant human Factor VIIa was added to the cells at a final concentration of 10 nM. One set of cells did not have Factor VIIa added as a negative control. The cells were incubated for 2 hours at 37°C and washed with HEPES buffer (10 mM HEPES, 137 mM NaCl, 4 mM KCl, 5 mM CaCl₂, 11 mM glucose, 0.1% BSA). After washing, cells were incubated for 5 min with 50 µl per well of 200 nM plasma-purified human Factor X in a Tris-buffered saline supplemented with 5 mM CaCl₂. Twenty-five microliters of 0.5 M EDTA and 25 µl of an 800 µM solution of S-2222 chromogenic substrate (Kabi Pharmacia, Franklin, OH) were added to each well. The plates were incubated for 40 min at room temperature, then analyzed at 405 nm using a THERMO MAX Microplate Reader (Molecular Devices, Menlo Park, CA).

Table 6 shows an increase in absorbance for the Factor VIIa treated wells as compared to the control wells (no Factor VIIa added). The increase in absorbance is a direct measurement of the level of Factor Xa generated in the wells and its subsequent cleavage of the chromogenic substrate, releasing the chromophore. The data also demonstrate that the level of chromogenic activity in cells pretreated with either PDGF-BB or 10% fetal calf serum was higher than unstimulated cells.

**Table 6**

| Test Sample | OD₄₀₅ |
|---|---|
| Control | 0.043 |
| Intrinsic | 0.247 |
| PDGF-BB | 0.360 |
| 10% FCS | 0.342 |

These results clearly show there is a Factor VIIa-dependent activation of Factor X to Factor Xa on the cell surface of rat vascular smooth muscle cells.

### Example VII

### Inhibition of cell-surface chromogenic

### activity by DEGR-Factor VIIa.

Rat vascular smooth muscle cells were plated into 96-well culture dishes as described above. The cells were cultured for 72 hours in serum free media as described above and treated with the addition of 10% fetal calf serum for 6 hours to stimulate tissue factor expression. After stimulation buffer only (control), 10 nM Factor VIIa, or 10 nM Factor VIIa + 100 nM DEGR-Factor VIIa was added to each well. The cells were incubated for 2 hours at 37°C, then washed with HEPES buffer. After washing, the cells were incubated for 5 minutes with 50 µl per well of 200 nM Factor X in Tris-buffered saline supplemented with 5 mM CaCl₂. Twenty-five microliters of 0.5 M EDTA and 25 µl of S-2222 (800 µM) chromogenic substrate (Kabi Pharmacia) were added to each well. The cells were incubated at room temperature for 40 minutes. Chromogenic activity was analyzed at 405 nm as described above.

Table 7 shows stimulation of chromogenic activity in the wells treated with Factor VIIa only, and inhibition of stimulation when DEGR-Factor VIIa was coincubated with the Factor VIIa. These results demonstrate that DEGR-Factor VIIa acts as a competitive antagonist for Factor VIIa binding, thereby inhibiting the activation of Factor X to Factor Xa and the subsequent cleavage of the S-2222 chromogen.

**Table 7**

| Test Sample | OD₄₀₅ |
|---|---|
| Control | 0.035 |
| Factor VIIa | 0.342 |
| Factor VIIa + DEGR-Factor VIIa | 0.073 |

### Example VIII

### Dose dependent inhibition by DEGR-Factor VIIa of cell surface chromogenic activity on rat smooth muscle cells.

Rat vascular smooth muscle cells were plated into 96-well culture dishes at 4,000 cells per well in growth medium supplemented with 1% fetal calf serum (as in Table 4 without 10% fetal calf serum). After 5 days the medium was removed, and either increasing concentrations of Factor VIIa alone or 10 nM Factor VIIa with increasing concentrations of DEGR-Factor VIIa were added to the cells. The cells were incubated with the Factor VII mixtures for 2 hours at 37°C. After incubation, the cells were washed and incubated with 50 µl of 200 nM Factor X in tris buffered saline for 5 minutes at room temperature. Each well had 25 µl of 0.5 M EDTA and 25 µl of 800 µM S-2222 (Kabi Pharmacia) added to it, and the plates were incubated for 40 minutes at room temperature. Chromogenic activity was analyzed at 405 nm in a microplate reader as described above.

Table 8 shows a dose-dependent increase in chromogenic activity with increasing amounts of Factor VIIa added to the wells. When the mixture of DEGR-Factor VIIa with 100 nM Factor VIIa was added to the cells (Table 9) there was a dose dependent inhibition in chromogenic activity. A 1:1 molar ratio of DEGR-Factor VIIa:Factor VIIa inhibited approximately 95% of the chromogenic activity. These data suggest that DEGR-Factor VIIa has a significantly higher affinity for cell-surface tissue factor than native Factor VIIa. If DEGR-Factor VIIa and Factor VIIa had equal affinity for binding tissue factor then the level of inhibition observed when the two molecules were added to the cells in an equal molar ratio would not have been as high.

**Table 8**

| Factor VIIa Conc. (nM) | OD₄₀₅ |
|---|---|
| 0.10 | 0.005 |
| 0.39 | 0.025 |
| 1.56 | 0.058 |
| 6.25 | 0.111 |
| 25.00 | 0.154 |
| 100.00 | 0.208 |

Table 9 shows the dose dependent inhibition of Factor Xa chromogenic activity on rat smooth muscle cells by DEGR-Factor VIIa. Increasing concentrations of DEGR-Factor VIIa were coincubated with 100 nM Factor VIIa, and the Factor Xa chromogenic activity determined using chromogenic substrate S-2222.

**Table 9**

| DEGR-Factor VIIa Conc. (nM) | OD₄₀₅ |
|---|---|
| 0.00 | 0.208 |
| 0.39 | 0.176 |
| 1.56 | 0.116 |
| 6.25 | 0.073 |
| 25.00 | 0.026 |
| 100.00 | 0.014 |

### Example IX

### Inhibition of Factor Xa generation by DEGR-Factor VIIa in a soluble tissue factor assay.

The conversion of Factor X to Factor Xa using purified recombinant soluble tissue factor was established using a chromogenic assay. Tissue factor was expressed and purified from Saccharomyces cerevisiae (Shigematsu et al., J. Biol. Chem. 267:21329-21337, 1992). Soluble tissue factor was purified and characterized by Dr. Walt Kisiel (University of New Mexico, Albequerque, NM). A reaction mixture containing 65.9 µl of soluble tissue factor (2.2 µM), 29.0 µl of PCPS (1 mM, Sigma, St. Louis, MO), 29.5 µl human Factor X (4.1 µM), 2.77 ml Hank's buffer (25 mM Tris, pH 7.4, 150 mM NaCl, 2.7 mM KCl, 5 mM CaCl₂, 0.1% BSA) was prepared. Forty microliter of tissue factor/Factor X mixture, 25 µl Factor VIIa diluted with TBS and 25 µl of DEGR-Factor VIIa diluted with TBS were added to each well of a 96-well microtiter plate. A control using 40 1 of tissue factor/Factor X mixture; 25 µl Factor VIIa diluted with TBS, and 25 µl of TBS only was included. Ten microliters of S-2222 (4 mM) chromogenic substrate was added to the reaction mixture in the wells and incubated at room temperature for 2-10 minutes. Results were analyzed at 405 nm in a microplate reader as described above.

Determination of a standard curve for Factor VIIa activation of Factor X was made using increasing concentrations of Factor VIIa added in the absence of DEGR-Factor VIIa. The results, presented in Table 10, show that there is a dose-dependent increase in chromogenic activity with increasing amounts of Factor VIIa added to the reaction mixture. The simultaneous addition of varying amounts of DEGR-Factor VIIa and 100 nM Factor VIIa led to a dose dependent decrease in chromogenic activity (Table 11). These data demonstrate that DEGR-Factor VIIa acts as a competitive antagonist for native Factor VIIa binding to soluble tissue factor, and thereby inhibits the generation of Factor Xa as measured by the decrease in chromogenic activity towards the chromogenic substrate S-2222.

**Table 10**

| Stimulation of Factor Xa chromogenic activity with increasing concentrations of Factor VIIa added to soluble tissue factor. Changes in optical density were measured using chromogenic substrate S-2222. | |
|---|---|
| Factor VIIa Conc (nM) | OD₄₀₅ |
| 0.78 | 0.168 |
| 1.56 | 0.288 |
| 3.12 | 0.478 |
| 6.25 | 0.694 |
| 12.50 | 0.764 |
| 25.00 | 0.790 |
| 50.00 | 0.738 |
| 100.00 | 0.770 |

**Table 11**

| Inhibition of Factor Xa chromogenic activity by the addition of DEGR-Factor VIIa to soluble tissue factor in the presence of native Factor VIIa is measured. Changes in optical density were measured using the chromogenic substrate S-2222. |
|---|
| DEGR-Factor VIIa Conc. (nM)OD₄₀₅ |
| 0.810 |
| 50.750 |
| 100.609 |
| 200.296 |
| 400.167 |
| 800.083 |
| 1600.055 |

### Example X

### Inhibition of coagulation by DEGR-Factor VIIa.

Standard clotting assays to monitor the effect of DEGR-Factor VIIa on clotting time were prepared as follows: 100 µl of normal baboon plasma, collected with sodium citrate as anticoagulant, was added to 100 µl of varying concentrations of DEGR-Factor VIIa diluted in TBS (20 mM Tris, pH 7.4, 150 mM NaCl). The samples were mixed and briefly incubated at 37°C. The samples were added to an Electra 800 Automatic Coagulation Timer (Medical Laboratories Automation, Pleasantville, NY). After incubation, 200 µl of a tissue factor preparation containing 25 mM CaCl₂ was added to the DEGR-Factor VIIa preparations. A tissue factor preparation was made as a saline extract of baboon brain that from freshly frozen brain tissue and characterized for its ability to initiate coagulation in baboon plasma. A concentration of tissue factor that gave a clotting time of about 40 seconds was selected.

The data, presented in Table 12, demonstrates a dose-dependent increase in clotting time due to the addition of DEGR-Factor VIIa. A dose as low as 1 µg/ml of DEGR-Factor VIIa in plasma resulted in a significant increase in clotting time.

**Table 12**

| Dose dependent increase in clotting time due to DEGR-Factor VIIa. | |
|---|---|
| DEGR-Factor VIIa (µg/ml plasma) | Clotting time (seconds) |
| 0 | 40.7 |
| 0.5 | 46.2 |
| 1.0 | 50.8 |
| 2.5 | 64.5 |
| 5.0 | 108.1 |
| 10.0 | 158.4 |

### Example XI

### Inhibition of platelet accumulation

### with DEGR-Factor VIIa.

DEGR-Factor VIIa was analyzed for its ability to inhibit platelet accumulation at sites of arterial thrombosis due to mechanical injury in non-human primates. A model of aortic endarterectomy was utilized in baboons, essentially as described by Lumsden et al. (Blood 81:1762-1770, 1993). A section of baboon aorta 1-2 cm in length was removed, inverted and scraped to remove the intima of the artery and approximately 50% of the media. The artery was reverted back to its correct orientation, cannulated on both ends and placed into an extracoporeal shunt in a baboon, thereby exposing the mechanically injured artery to baboon blood via the shunt. Just prior to opening of the shunt to the circulating blood, ¹¹¹In-labeled autologous platelets were injected intravenously into the animal. The level of platelet accumulation at the site of the injured artery was determined by real-time gamma camera imaging.

Evaluation of DEGR-Factor VIIa for inhibition of platelet accumulation was done using bolus injections of DEGR-Factor VIIa or saline control and were given just prior to the opening of the shunt. The injured arteries were measured continuously for 60 minutes. A dose of 0.005 mg/kg of DEGR-Factor VIIa inhibited platelet accumulation. At a 1.0 mg/kg bolus injection, approximately 90% of platelet accumulation was inhibited at 1 hour post drug administration.

These data show that inhibition of tissue factor with DEGR-Factor VIIa can significantly inhibit the development of platelet-rich thrombi in a nonhuman primate model of acute vascular injury.

It is evident from the foregoing that compositions of Factor VII or VII having modified catalytic sites are provided which are able to bind tissue factor yet are substantially unable to activate Factors X and IX. As modified Factor VII acts specifically to interrupt the clotting cascade without degrading or consuming clotting factors, it can be expected that administration of modified Factor VII preparations will be accompanied by fewer undesirable side effects than experienced with current therapies. Further, the modified Factor VII described herein may readily be produced by recombinant means. Thus efficacy, convenience and economics of lower dosages and less frequent administration, and a relative lack of toxicity are among the advantages conferred.

## Claims

1. Use of Factor VII having at least one modification in its catalytic center, which modification substantially inhibits the ability of the modified Factor VII to activate plasma Factor X or IX, in the manufacture of a medicament for the inhibition of vascular restenosis.

2. The use according to claim 1, wherein the modification comprises reaction of the Factor VII with a serine protease inhibitor.

3. The use according to claim 2, wherein the protease inhibitor is an organophosphor compound, a sulfanyl fluoride, a peptide halomethyl ketone, or an azapeptide.

4. The use according to claim 3, wherein the protease inhibitor is a peptide halomethyl ketone selected from D-Phe-Pro-Arg chloromethyl ketone or Dansyl-Glu-Gly-Arg chloromethyl ketone.

5. Use of Factor VII having at least one modification in its catalytic center, which modification substantially inhibits the ability of the modified Factor VII to activate plasma Factor X or IX, in the manufacture of a medicament for inhibiting platelet deposition.

6. The use according to claim 5, wherein the modification comprises reaction of the Factor VII with a serine protease inhibitor.

7. The use according to claim 5, wherein the protease inhibitor is an organophosphor compound, a sulfanyl fluoride, a peptide halomethyl ketone, or an azapeptide.

8. The use according to claim 7, wherein the protease inhibitor is a peptide halomethyl ketone selected from D-Phe-Pro-Arg chloromethyl ketone or Dansyl-Glu-Gly-Arg chloromethyl ketone.

9. The use according to claim 5, wherein the Factor VII modification comprises at least one amino acid substitution, insertion, or deletion in a catalytic triad of Ser, Asp, and His.

## Patentansprüche

1. Verwendung von Faktor VII, der wenigstens eine Modifikation in seinem katalytischen Zentrum hat, die wesentlich die Fähigkeit des modifizierten Faktors VII inhibiert, Plasma-Faktor X oder IXₓ zu aktivieren, bei der Herstellung eines Medikaments zur Unterdrückung der Gefäßrestenose.

2. Verwendung nach Anspruch 1, wobei die Modifikation die Reaktion des Faktors VII mit einem Serinproteaseinhibitor umfasst.

3. Verwendung nach Anspruch 2, wobei der Proteaseinhibitor eine Organo-Phosphorverbindung, ein Sulfanylflurid, ein Peptid-Halomethylketon oder ein Azapeptid ist.

4. Verwendung nach Anspruch 3, wobei der Proteaseinhibitor ein Peptid-Halomethyl-Keton ist, das aus D-Phe-Pro-Arg-Chloromethyl-Keton oder Dansyl-Glu-Gly-Arg-Chloromethyl-Keton gewählt ist.

5. Verwendung von Faktor VII, der wenigstens eine Modifikation in seinem katalytischen Zentrum hat, die wesentlich die Fähigkeit des modifizierten Faktors VII inhibiert, Plasma-Faktor X oder IXₓ zu aktivieren, bei der Herstellung eines Medikaments zur Unterdrückung der Blutblättchenablagerung.

6. Verwendung nach Anspruch 5, wobei die Modifikation die Reaktion des Faktors VII mit einem Serinproteaseinhibitor umfasst.

7. Verwendung nach Anspruch 5, wobei der Proteaseinhibitor eine Organo-Phosphorverbindung, ein Sulfanylflurid, ein Peptid-Halomethylketon oder ein Azapeptid ist.

8. Verwendung nach Anspruch 7, wobei der Proteaseinhibitor ein Peptid-Halomethylketon ist, das aus D-Phe-Pro-Arg-Chloromethyl-Keton oder Dansyl-Glu-Gly-Arg-Chloromethyl-Keton gewählt ist.

9. Verwendung nach Anspruch 5, wobei die Modifikation des Faktors VII wenigstens eine Aminosäuresubstitution, -einschiebung oder -deletion in der katalytischen Triade Ser, Asp und His umfasst.

## Revendications

1. Utilisation de facteur VII présentant au moins une modification dans son centre catalytique, laquelle modification inhibe pratiquement l'aptitude du facteur VII modifié à activer le facteur plasmatique X ou XI, dans la fabrication d'un médicament pour l'inhibition de la resténose vasculaire.

2. Utilisation selon la revendication 1, dans laquelle la modification comprend la réaction du facteur VII avec un inhibiteur de sérine protéase.

3. Utilisation selon la revendication 2, dans laquelle l'inhibiteur de protéase est un composé organophosphoré, un fluorure de sulfanyle, une peptide-halogénométhylcétone ou un azapeptide.

4. Utilisation selon la revendication 3, dans laquelle l'inhibiteur de protéase est une peptlde-halogénométhylcétone choisie parmi la D-Phe-Pro-Arg-chlorométhylcétone et la dansyl-Glu-Gly-Arg-chlorométhylcétone.

5. Utilisation de facteur VII présentant au moins une modification dans son centre catalytique, laquelle modification inhibe pratiquement l'aptitude du facteur VII modifié à activer le facteur plasmatique X ou XI, dans la fabrication d'un médicament pour l'inhibition du dépôt des plaquettes.

6. Utilisation selon la revendication 5, dans laquelle la modification comprend la réaction du facteur VII avec un inhibiteur de sérine protéase.

7. Utilisation selon la revendication 5, dans laquelle l'inhibiteur de protéase est un composé organophosphoré, un fluorure de sulfanyle, une peptide-halogénométhylcétone ou un azapeptide.

8. Utilisation selon la revendication 7, dans laquelle l'inhibiteur de protéase est une peptide-halogénométhylcétone choisie parmi la D-Phe-Pro-Arg-chlorométhylcétone et la dansyl-Glu-Gly-Arg-chlorométhylcétone.

9. Utilisation selon la revendication 5, dans laquelle la modification du facteur VII comprend au moins un remplacement, une insertion ou une délétion d'aminoacide dans un triade catalytique de Ser, Asp et His.
